# EUROPEAN PATENT APPLICATION

(11) **EP 1 870 013 A1**
(43) Date of publication of application: **26.12.2007**
(21) Application number: 06713613.5
(22) Date of filing: 13.02.2006
(51) Int. Cl.: A61B 1/00, G09G 5/00, A61B 5/07, A61B 5/00

(54) **IMAGE DISPLAY DEVICE**

(30) Priority: 14.04.2005 JP 2005117523
(71) Applicant: OLYMPUS MEDICAL SYSTEMS CORP., Shibuya-ku Tokyo 151-0072 (JP)
(72) Inventor: KIMOTO, Seiichiro c/o Olympus Medical Systems Corp., Shibuya-ku, Tokyo 151-0072 (JP)
(74) Representative: von Hellfeld, Axel
(86) International application number: PCT/JP2006/302471
(87) International publication number: WO 2006/112116

(57) **Abstract**

An object of the present invention is to realize an image display apparatus which is capable of realizing a real-time display of an image based on a radio signal, and of coping with a case where a viewer such as a doctor overlooks an image. The image display apparatus includes a storage unit 11 which retains an electric signal and the like output from a switching unit 10 for a predetermined time; and an image data generator 12 which generates predetermined image data based on the electric signal output from the switching unit 10 or the electric signal stored in the storage unit 11. Since the image display apparatus has a configuration that the image data generator 12 not only receives the electric signal directly from the switching unit 10, but also receives the electric signal to which a predetermined time lag is given in the storage unit 11, thereby enabling a display of a real-time image and a past image to which the predetermined time lag is given with respect to the real-time image.

## Description

### TECHNICAL FIELD

The present invention relates to an image display apparatus capable of sequentially displaying a plurality of images based on an electric signal which is sequentially generated at least via a receiving process on a predetermined radio signal.

### BACKGROUND ART

In recent years, a swallowable capsule endoscope has been proposed in a field of endoscopes. The capsule endoscope is provided with an imaging function and a radio communication function. The capsule endoscope travels through inside a body cavity, for example, through inside organs such as the stomach and the small intestine, during a period from when the capsule endoscope is inserted from a mouth of a subject for an observation (examination) until when the capsule endoscope is naturally excreted, while following peristaltic motion of the organs. The capsule endoscope has a function of capturing an intra-subject image for, for example, every 0.5 seconds.

While the capsule endoscope travels inside the body cavity, image data captured by the capsule endoscope in the subject body is sequentially transmitted to the outside of the subject by a radio communication to be stored in a memory provided in a receiving device. The subject carrying such a portable receiving device can freely move during the period from when the capsule endoscope is swallowed until when the capsule endoscope is excreted (see Patent Document 1, for example).

In such a capsule endoscope system, it is a common practice that the image data stored in the receiving device is transferred to a work station and the like after the capsule endoscope completes the series of imaging operation, and after-the-fact perusal of the images are performed. However, since a doctor or the like has a strong demand for a real-time perusal of the captured images with respect to a site of interest and the like, a system including a compact and portable image display apparatus which displays images in real-time based on a radio signal transmitted from the capsule endoscope, has been proposed.

The conventional image display apparatus can be electrically connected to a receiving device, and includes a predetermined signal processor and a compact display screen in its simplest configuration. With such a configuration, the simple image display apparatus can receive a signal to which a receiving process is performed in the receiving device, and can display the images captured by the capsule endoscope on the small display screen after performing a predetermined process based on the received signal, so that the doctor or the like can perform a real-time perusal of the images.

Patent Document 1: Japanese Patent Application Laid-Open No. 2003-19111

### DISCLOSURE OF INVENTION

### PROBLEM TO BE SOLVED BY THE INVENTION

However, the conventional image display apparatus constituting the capsule endoscope system has a problem of having a difficulty in performing satisfactory perusal of the images captured by the capsule endoscope. Specifically, when the doctor or the like misses an image with respect to the site of interest because of the display of the real-time images, it is difficult to display the image again, and therefore impossible to take advantage of having incorporated the image display apparatus in the system. On the other hand, it is neither preferable that the image display apparatus is configured to read out the data recorded in the receiving device because such a configuration leads to making the process cumbersome, nor that the image display apparatus is configured to have a storage unit of large-capacity which enables recording all electric signals received from the receiving device and reading out the data of the past images as necessary because such a configuration leads to making the size of the image display apparatus grow. Specifically, the capsule endoscope is, for example, configured to perform the imaging operation twice per second and travel the inside of the subject at such an imaging rate over a several hours. Therefore, it is necessary to have the storage unit of extremely large-capacity for storing electric signals corresponding to all images, and thereby it is not realistic to incorporate such a storage unit into the compact image display apparatus.

In view of the foregoing, an object of the present invention is to realize an image display apparatus capable of achieving a real-time display of the images based on a radio signal transmitted from a body-insertable device such as a capsule endoscope, and also capable of coping with the case where the viewer such as the doctor or the like may overlook images.

### MEANS FOR SOLVING PROBLEM

To solve the problems described above and to achieve the object, an image display apparatus according to claim 1, which is capable of sequentially displaying a plurality of images based on an electric signal sequentially generated via at least a receiving process on a predetermined radio signal, includes a display unit that can display a real-time image as an image to be displayed via a sequential process in accordance with a generation of the electric signal, and a past image as an image to be displayed in a predetermined time lag after the generation of the electric signal.

In the invention according to claim 1, not only the real-time image to be displayed in real-time via a sequential process on the obtained electric signal but also the past image with a predetermined time lag from the real-time can be displayed. Thus, even when a user misses a necessary image for some reason during a perusal of the real-time, it is possible to refer to and peruse the past image to check the content thereof.

The image display apparatus according to the invention as set forth in claim 2, further includes a buffer memory unit that, after the generation of the electric signal, retains one of the electric signal and image data which is generated based on the electric signal for a predetermined time to output thereafter, wherein the display unit displays the past time image based on one of the electric signal and the image data output from the buffer memory unit.

In the image display apparatus according to the invention as set forth in claim 3, the display unit displays the real-time image and the past image on the same screen at the same time.

In the image display apparatus according to the invention as set forth in claim 4, the display unit has a main image area and a subimage area which is smaller than the main image area on the display screen, and displays an image which is selected among the plurality of images displayed at the same time, in the main image area.

In the image display apparatus according to the invention as set forth in claim 5, the electric signal is compressed data on which a predetermined compression process is performed, and the image display apparatus further includes a data expander that performs an expansion process with respect to the compressed data when image data is generated based on the electric signal.

The image display apparatus according to the invention as set forth in claim 6, the display unit performs a partial display by appropriately thinning out the real-time images depending on a process speed in the image display apparatus, while all images corresponding to the electric signal are displayed with respect to the past image.

### EFFECT OF THE INVENTION

The image display apparatus according to the present invention is capable of displaying not only the real-time image which is displayed in real-time via a sequential process on the obtained electric signal but also the past image to which a predetermined time lag from the real-time is given. Thus, the image display apparatus according to the present invention is advantageous in that, even when a user misses a necessary image for some reason during the perusal of the real-time, it is possible to refer to and peruse the past image to check the content thereof.

### BRIEF DESCRIPTION OF DRAWINGS

FIG. 1 is a schematic diagram of an embodiment of a usage of an image display apparatus;
FIG. 2 is a schematic block diagram of a configuration of the image display apparatus according to a first embodiment;
FIG. 3 is a schematic diagram of a configuration of a buffer memory provided to the image display apparatus;
FIG. 4 is a schematic diagram of a display screen of a display unit provided to the image display apparatus;
FIG. 5 is a schematic block diagram of a configuration of an image display apparatus according to a second embodiment; and
FIG. 6 is a schematic diagram of a display form of real-time images and past time images.

### EXPLANATIONS OF LETTERS OR NUMERALS

- 1: IMAGE DISPLAY APPARATUS
- 2: SUBJECT
- 3: CAPSULE ENDOSCOPE
- 4: RECEIVING DEVICE
- 5a to 5h: RECEIVING ANTENNAS
- 7: INPUT TERMINAL
- 8: RECEIVING ANTENNA
- 9: DEMODULATOR
- 10: SWITCHING UNIT
- 11: STORAGE UNIT
- 12: IMAGE DATA GENERATOR
- 13: SCREEN IMAGE GENERATOR
- 14: DISPLAY UNIT
- 15: OPERATING UNIT
- 16: CONTROL UNIT
- 17: DISPLAY CONTROLLER
- 18: BUFFER MEMORY
- 19: STORAGE AREA
- 21: DISPLAY SCREEN
- 22: MAIN IMAGE AREA
- 23a: SUBIMAGE AREA
- 23b: SUBIMAGE AREA
- 24: REAL-TIME IMAGE
- 25: PAST IMAGE
- 26: CAPTURED IMAGE
- 28: IMAGE DISPLAY APPARATUS
- 29: DATA EXPANDER
- 30: IMAGE DATA GENERATOR
- 31: CONTROL UNIT
- 32: DISPLAY CONTROLLER

### BEST MODE(S) FOR CARRYING OUT THE INVENTION

Best modes of an image display apparatus according to the present invention (simply referred to as "embodiments" hereinbelow) will be explained below. It should be noted that the accompanying drawings are merely schematic, and the size and the like of each portion may be different from the reality.

(First embodiment) An image display apparatus according to a first embodiment will be explained. An embodiment of a usage of the image display apparatus according to the first embodiment will first be explained, and then a configuration, effect, and the like of the image display apparatus will be explained.

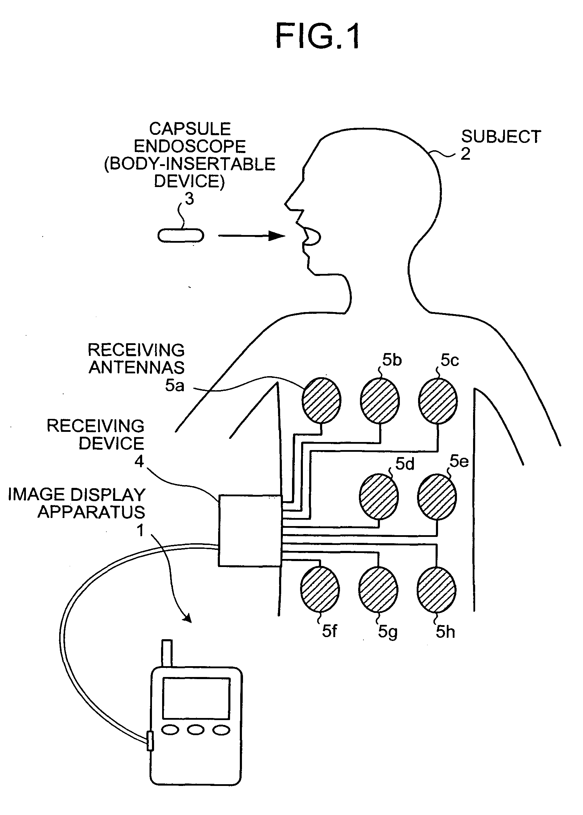

FIG. 1 is a schematic diagram of a usage of the image display apparatus according to the first embodiment. As shown in FIG. 1, an image display apparatus 1 according to the first embodiment, which is for displaying an image based on a radio signal transmitted from a capsule endoscope 3 inserted into a subject 2, is used for the purpose of displaying multiple images captured by the capsule endoscope 3 by being electrically connected to a receiving device 4 as necessary via a predetermined cable and the like, or by independently receiving a radio signal from the capsule endoscope 3.

The capsule endoscope 3 functions as a transmission source of a radio signal. Specifically, the capsule endoscope 3 is inserted through an oral cavity of the subject 2 into the inside of the subject 2, and functions to repeat an imaging operation at predetermined time intervals by a built-in imaging mechanism and to wirelessly transmit multiple pieces of image data obtained by the imaging operation to the outside of the subject 2 sequentially, for example.

The receiving device 4 receives the radio signal transmitted from the capsule endoscope 3 via an antenna selected among receiving antennas 5a to 5h, performs a predetermined process to generate predetermined image data, and records the generated image data, for its primary functions. The recorded image data is output to a predetermined work station or the like after the completion of using the capsule endoscope 3, and the images are displayed on a monitor of the work station or the like for a diagnosis to be performed by a doctor or the like.

The receiving device 4 is used in a state of being carried by the subject 2 as shown in FIG. 1. Such a usage is employed for preventing an action of the subject 2 from being restricted while the capsule endoscope is in the subject 2. In other words, though the receiving device 4 needs to keep receiving radio signals transmitted for a several hours to several tens of hours during the travel of the capsule endoscope 3 inside the subject 2, if the subject 2 is required to be in a hospital for such a long time, the convenience of using the capsule endoscope 3 may be impaired. Therefore in the first embodiment, the receiving device 4 is downsized to a degree of being portable, so that the degree of freedom in the action of the subject 2 can be secured even while the capsule endoscope 3 is in the subject 2, and the burden of the subject 2 can be reduced.

Next, the image display apparatus 1 will be explained. The image display apparatus according to the first embodiment is capable of displaying images of the inside of the subject 2 captured by the capsule endoscope 3 by sequentially changing one to another in an order of imaging, i.e., an image display in a pseudo-moving mode. The image display apparatus according to the first embodiment, in addition to the basic function, has a configuration including a display unit that displays a real-time image as the first image just after the imaging; a past image as an image to be displayed in a predetermine time lag after the imaging time compared with the real-time image; and a captured image which is specified by a capturing process designated by a user.

Specifically, the image display apparatus 1 according to the first embodiment includes an input terminal 7 for receiving the electric signal output from the receiving device 4; a receiving antenna 8 for receiving the radio signal transmitted from the capsule endoscope 3; a demodulator 9 which performs a demodulating process on the radio signal received via the receiving antenna 8; and a switching unit 10 which selects and outputs one of the electric signal output from the demodulator 9 and the electric signal received from the receiving device 4 via the input terminal 7. The image display apparatus 1 also includes a storage unit 11 which stores the electric signal and the like output from the switching unit 10; an image data generator 12 which generates predetermined image data based on the electric signal output from the switching unit 10 or the electric signal stored in the storage unit 11; a screen image generator 13 which generates a screen image to be displayed on a screen by combining a plural types of image data generated by the image data generator 12; and a display unit 14 that displays the screen image generated by the screen image generator 13. The image display apparatus 1 further includes an operating unit 15 for inputting an instruction which designates a display form and the like of the display unit 14; and a control unit 16 which performs a predetermined control based on information input via the operating unit 15.

The receiving antenna 8 and the demodulator 9 each function as an example of a receiving mechanism in the scope of claims, and has a function of performing a receiving process directly on the radio signal transmitted from the capsule endoscope 3. From the view point for enabling an image display based on the radio signal transmitted from the capsule endoscope 3, it is sufficient to have one of the input terminal 7 and the pair of the receiving antenna 8 and the demodulator 9. However in the first embodiment, the image display apparatus has a receiving mechanism in itself from the view point for enabling an image display in an independent image display apparatus.

The switching unit 10 is for outputting one of the electric signal received via the input terminal 7 and the electric signal output from the demodulator 9 to the storage unit 11 and the image data generator 12. In the first embodiment as described, there are two cases as a mode of obtaining the electric signal based on the radio signal transmitted from the capsule endoscope 3, i.e., a case where the electric signal is received from the receiving device 4 via the input terminal 7; and a case where the electric signal is obtained via the receiving antenna 8 and the demodulator 9. Therefore, the switching unit 10 is configured to select one of the two modes of electric signals based on a control by the control unit 16 to switch between the electric signals to be output.

The image data generator 12 has a function of generating image data based on the input electric signal. Specifically, the image data generator 12 has a function of generating image data corresponding to a real-time image based on the electric signal input form the switching unit 10, and generating image data corresponding to a past image based on the electric signal retained in the storage unit 11 for a predetermined time. Here, the "real-time image" is an image captured by the capsule endoscope 3, and represents an image having as small time lag as possible between the imaging time by the capsule endoscope 3 and the display time by the display unit 14 via a sequential process on the electric signal input from the switching unit 10. The "past image" is an image captured by the capsule endoscope 3, and represents an image having a predetermined time lag between the imaging time by the capsule endoscope 3 and the display time by the display unit 14. In the first embodiment, in addition to these images, the image data generator 12 can also generate image data corresponding to a captured image as an image specially designated by the user.

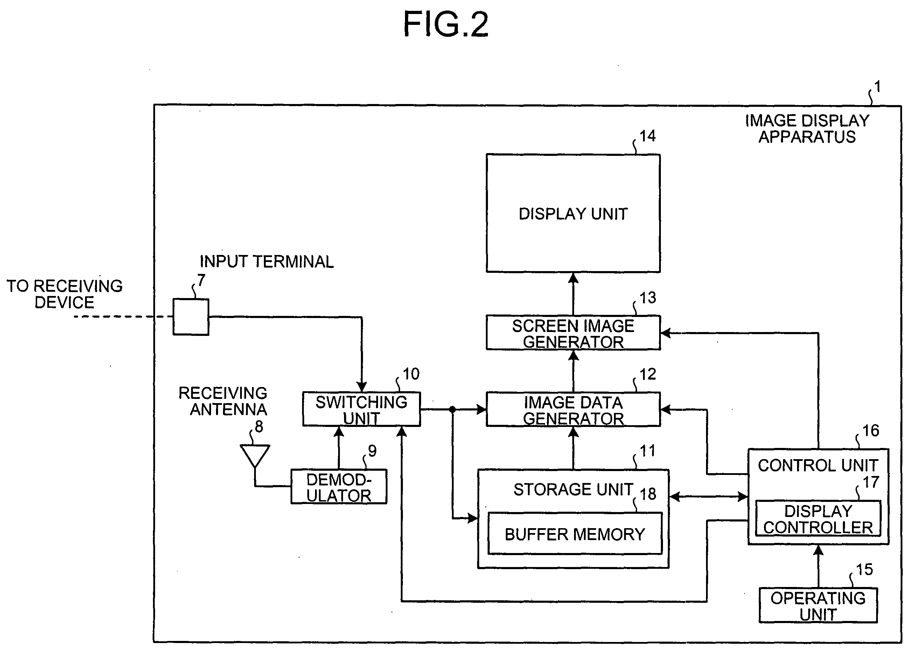

The screen image generator 13 is for generating a screen image to be displayed on the display unit 14 based on the image data output from the image data generator 12. As described, the image data generator 12 has a function of generating a screen image in which the real-time image, past image, and the captured image are arranged in a predetermined form, and of outputting the image data corresponding to the screen image to the control unit 16. In the example shown in FIG. 2, the image data generator 12 and the screen image generator 13 each are separate and independent components for easy understanding. However, the image data generator 12 and the screen image generator 13 may be formed integrally.

The operating unit 15 is for inputting operation information of a display form and the like of the real-time image and the past image on the display unit 14. Specifically, the operating unit 15 has a configuration capable of inputting information necessary for an image display, such as a setting of the display area of the real-time image, past image, and the like on the screen. The information input via the operating unit 15 is transferred to the control unit 16, and the control unit 16 controls each component to perform a predetermined process based on the information input via the operating unit 15. The operating unit 15 may be, for example, configured to be capable of instructing to change a display speed, to stop the changeover of the images, and the like with respect to past time images which are displayed as a pseudo-moving image by consecutively changing a plurality of images. Alternatively, the operating unit 15 may be configured to be capable of changing the imaging time lag between the real-time image and the past image.

The control unit 16 has a function of performing a general driving control with respect to the components of the image display apparatus 1, and controlling the storage unit 11, the image data generator 12, and the like according to the content of the image to be displayed on the display unit 14. Specifically, the control unit 16 has a function of controlling the switching mode of the switching unit 10 based on the information input via the operating unit 15, and controlling the form of the images to be displayed on the display unit 14 via the built-in display controller 17.

Next, the storage unit 11 will be explained. The storage unit 11 has a function of storing a parameter and the like for display setting, and storing the electric signal corresponding to the captured image and the electric signal corresponding to the past image for a predetermined time. Specifically, the storage unit 11 has a function of storing the electric signal corresponding to the past image in the buffer memory 18 provided therein.

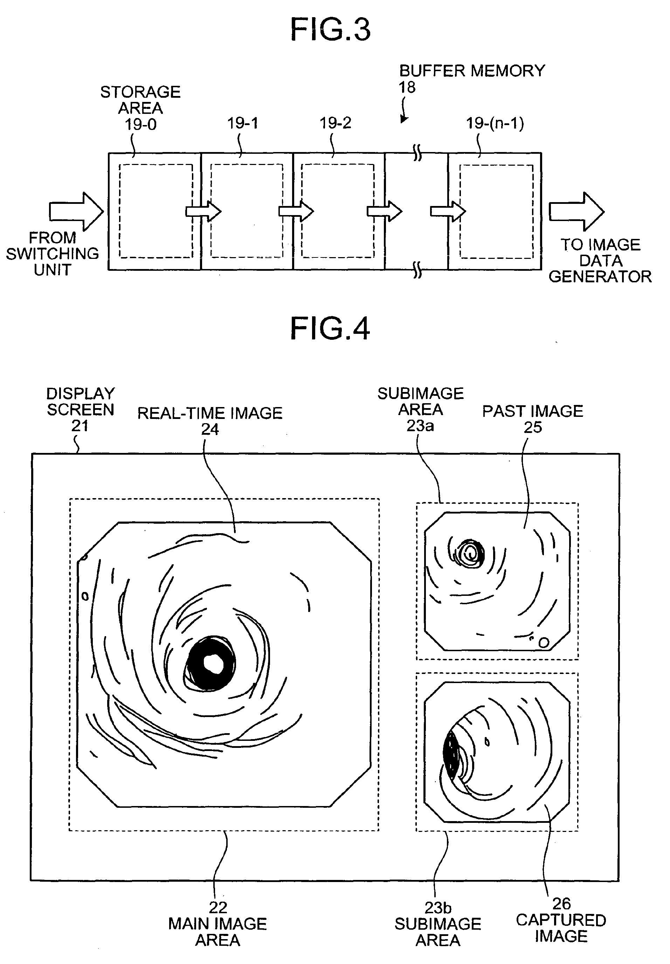

FIG. 3 is a schematic diagram for explaining information to be stored in the buffer memory 18. As shown in FIG. 3, the buffer memory 18 has storage areas 19-0 to 19- (n-1), the number of the storage areas corresponding to the imaging time lag between the real-time image and the past image, and is formed to sequentially shift the storing electric signals in accordance with the time passage. In other words, when the imaging time of the real-time image to be displayed in the display unit 14 is set to t₁, the electric signal to be stored in the storage area 19-0 corresponds to the image captured at the time t₁ (i.e., the real-time image), the electric signal to be stored in the storage area 19-1 corresponds to the image captured at the time (t₁-Δt), and the electric signal to be stored in the storage area 19-(n-1) corresponds to the image imaged at the time {t₁-(n-1)Δt}.

The buffer memory 18 shifts the electric signals respectively stored in the storage areas 19-0 to 19-(n-2) to the subsequent storage areas, i.e., the storage areas 19-1 to 19- (n-1) in accordance with the timing when the electric signal corresponding to a new image to be displayed as a real-time image in accordance with the time passage is input to the image data generator 12. The electric signal corresponding to the new image is then stored in the storage area 19-0 which has just become an empty space, and the electric signal stored in the storage area 19-(n-1) is output to the image data generator 12.

Detailed explanation will be given below. At a timing when the real-time image captured at the time t₁ is changed to the real-time image captured at time t₂ (=t₁+Δt), the electric signals corresponding to images captured at times t₁ (=t₂-Δt) to {t₁-(n-2)Δt} (=t₂-(n-1) Δt) are stored in the storage areas 19-1 to 19-(n-1), respectively, and the electric signal corresponding to the image newly captured at the time t₂ is stored in the storage area 19-0, which has just become an empty space. The electric signal corresponding to the image captured at the time {t₁-(n-1)Δt} (=t₂-nΔt) stored in the storage area 19-(n-1) is output to the image data generator 12, converted to image data with respect to the past image by the image data generator 12, and output to the screen image generator 13.

As described, the buffer memory 18 has a function of outputting the electric signal corresponding to the image captured in a predetermined time past after the imaging time of the real-time image. Thus, the image data generator 12 receives the electric signal directly input from the switching unit 10 (for example, the electric signal corresponding to the image captured at the imaging time t₁), and the electric signal output from the buffer memory 18 (the electric signal corresponding to the image captured at the imaging time t₁-nΔt) almost at the same time. The image data generator 12 performs a predetermined process on each of the electric signals, and then outputs the image data corresponding to each of the real-time image and the past image which is captured in the time nΔt past after the real-time image, to the screen image generator 13.

Next, a display form of the display unit 14 based on the image data generated by the image data generator 12 will be explained. It is possible to set an arbitrary configuration for the display form of the real-time image, the past image, and the captured image, whose respective image data is generated by the image data generator 12. Here, the first embodiment employs a configuration of displaying all images on the same screen at the same time as explained below.

FIG. 4 is a schematic diagram of a display form of images on a display screen 21 of the display unit 14. As shown in FIG. 4, the display screen 21 is configured to have a main image area 22, and subimage areas 23a and 23b which are smaller than the main image area 22 in advance, in each of which an image is to be displayed. In other words, in the example shown in FIG. 4, a real-time image 24 as an latest image is displayed in the main image area 22, a past image 25 as an image captured in a predetermined time past after the imaging time of the real-time image is displayed in the subimage area 23a, and a captured image 26 as an image designated via an operation of the operating unit 15 is displayed in the subimage area 23b. The display form of the real-time image 24 and the like is not limited to what is shown in FIG. 4, and it is possible to change the place for display by inputting predetermined information via the operating unit 15. Specifically, the past image may be displayed in the main image area 22 and the real-time image may be displayed in the subimage area 23a by designating via the operating unit 15.

Next, advantages of the image display apparatus according to the first embodiment will be explained. First of all, the image display apparatus according to the first embodiment has a configuration capable of not only displaying the real-time image obtained by performing a sequential receiving process on the radio signal transmitted from the capsule endoscope 3, but also displaying the past image captured in a predetermined time past with respect to the real-time image. Thus, the image display apparatus according to the first embodiment is advantageous in that even when the user perusing the real-time image misses a given image, the user can easily grasp the content of the missed image by referring to the past image separately.

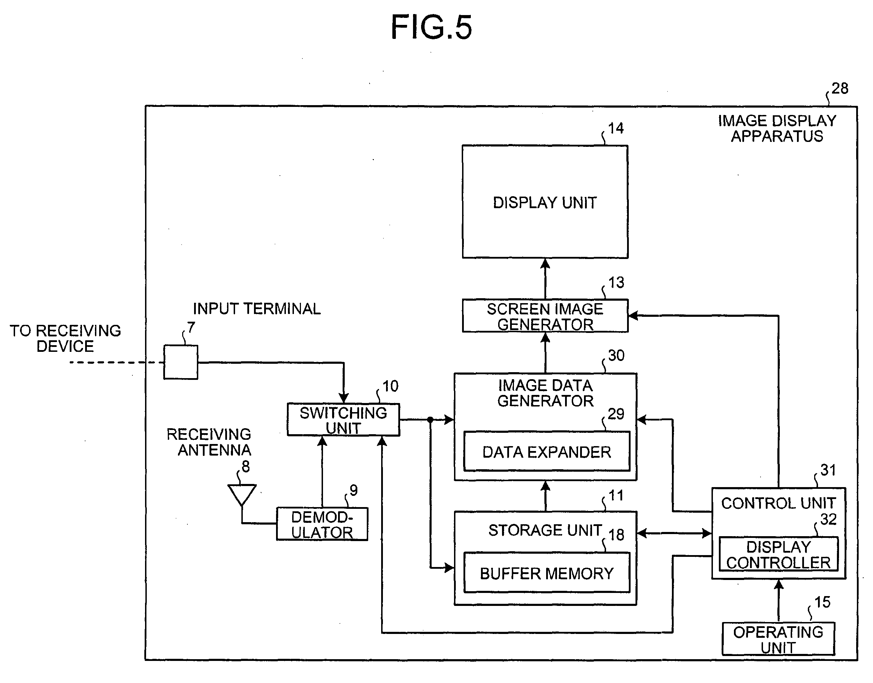

The image display apparatus according to the first embodiment employs a configuration of displaying the real-time image and the past image on the same screen of the display unit 14 at the same time as shown in FIG. 5. Therefore, even when the user misses a given image for some reason during the perusal of the real-time image, the user can refer to the past image by taking a changed glance thereto, resulting in an advantage that it is not necessary to perform complicated operation and the like.

In addition, there is another advantage thanks to displaying the real-time image and the past image at the same time. Specifically in the case of an image display apparatus which displays only the past image on the screen, it is impossible to grasp the content of the real-time image while referring to the past image though the real-time image changes from one to another constantly while referring to the past image. In contrast, the image display apparatus according to the first embodiment employs the configuration that the real-time image is kept displayed at the same time as displaying the past image, and thereby is advantageous in that the real-time image can be checked as referring to the past image.

The image display apparatus according to the first embodiment further employs the configuration of storing the electric signal, based on which the past image data is generated, in the buffer memory 18. Here, since the capsule endoscope 3 performs the imaging operation over a several hours to several tens of hours at an imaging speed for twice a second, it is necessary to prepare a large storage capacity when the electric signals corresponding to all images are to be stored. On the other hand, the buffer memory 18 employs a configuration of sequentially shifting the storing electric signals in accordance with the time passage, while having a given number of storage areas corresponding to the imaging time lag between the real-time image and the past image. Therefore, it is possible to suppress the storage capacity to as low value as possible, resulting in an advantage that the image display apparatus can be downsized.

(Second embodiment) Next, an image display apparatus according to a second embodiment will be explained. In the image display apparatus according to the second embodiment, an electric signal contained a radio signal transmitted from a capsule endoscope has a configuration that a predetermined compression process is performed on the electric signal obtained via the imaging operation to cope with the problem of a prolonged time for the data process.

FIG. 5 is a schematic block diagram of a configuration of the image display apparatus according to the second embodiment. As shown in FIG. 5, an image display apparatus 28 according to the second embodiment has a configuration including the input terminal 7; the receiving antenna 8; the demodulator 9; the switching unit 10; the storage unit 11; the screen image generator 13; the display unit 14; the operating unit 15; and the buffer memory 18, similarly to the image display apparatus according to the first embodiment. On the other hand, the image display apparatus 28 according to the second embodiment further includes an image data generator 30 which has a data expander 29 that performs a data expansion process in response to the compression-processed electric signal contained in the transmitted radio signal; a control unit 31 which controls components including the data expander 29; and a display controller 32 which performs a display control responding to the necessity of the data expansion process.

The data expander 29 is for performing a data expansion process on the electric signal input via the switching unit 10. From the view point of reducing the amount of information to be transferred, the electric signal contained in the radio signal transmitted from the capsule endoscope 3 is configured to be subject to the compression process, and the electric signal demodulated after the reception further needs to be subject to the expansion process in the first embodiment. Since the expansion process performed by the data expander 29 is added, an explanation will be given in the following as configured that the value for the amount of time required for the data process since the transmission of the radio signal from the capsule endoscope 3 until the display of the image on the display unit 14 is greater than the time interval in the imaging of the capsule endoscope 3.

Next, a display control of the display controller 32 to cope with the problem of prolonged time for the data process will be explained. As described above, in the image display apparatus according to the second embodiment, the value for the time required for the data process on the electric signal corresponding to each image is greater than the interval in imaging. For this reason, when the electric signal input in the same manner as the case of the first embodiment is sequentially processed to be displayed on the display unit 14 as the real-time image in the second embodiment, the time lag between the imaging time and the display time becomes increased gradually, and the real-time property of the display image is impaired. Therefore, the display controller 32 does not generate and display image data for every electric signal input via the switching unit 10, and performs data process and image display by appropriately thinning out the electric signals respectively corresponding to multiple images.

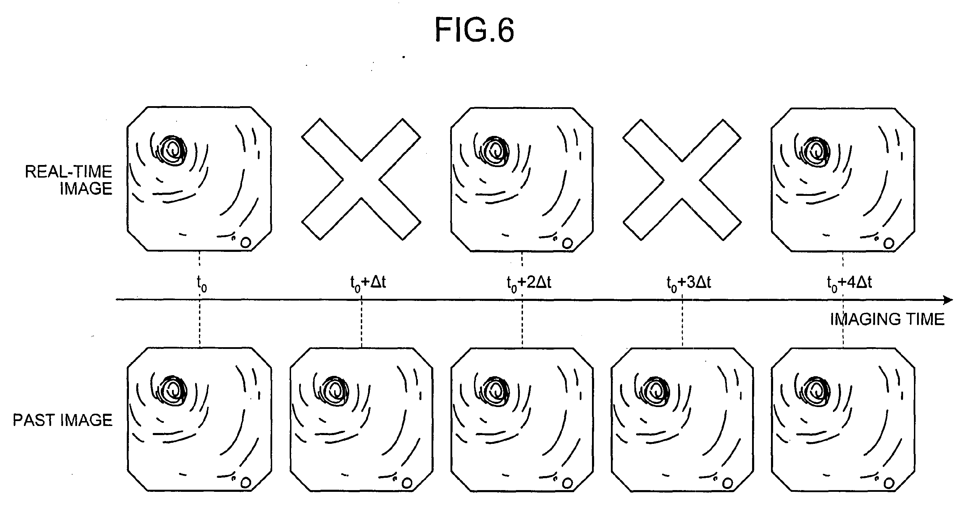

FIG. 6 is a schematic diagram for explaining a specific manner of the display control of the display controller 32 according to the second embodiment. In the case shown in FIG. 6, the time interval in imaging of the capsule endoscope 3 is set to be Δt, and the value for the time required for the process in the image display apparatus is set to be greater than the Δt.

As shown on the upper half in FIG. 6, the display controller 32, with respect to the real-time images, does not display all images which are sequentially captured in the order, to, t₀+Δt, t₀+2Δt..., and instead controls to display like to, t₀+2Δt... after thinning out the images. Such a partial display prevents a delay in the data process from occurring, and the real-time property from being impaired.

The image display apparatus according to the second embodiment can suppress the decrease in the amount of information to be displayed to the degree where there is no actual problem, even when the real-time images are displayed via the thinning-out. This is because the image display apparatus according to the second embodiment is configured to display the past images in the same manner as the first embodiment, i.e., the image display apparatus according to the second embodiment employs a configuration that all of the captured images are displayed in the display form for the past image as shown on the bottom half in FIG. 6, without thinning out the images for display. This is also because the past time image, different from the case of the real-time image, has a predetermined extra time before the image is displayed on the display unit 14 after the actual imaging, and thereby the image display apparatus can cope with an increase in the process time necessary for the expansion process. Accordingly, when an unusual site or the like is found during the perusal of the real-time image, the user such as the doctor can make more detailed diagnosis and the like separately by referring to the past images, i.e., all of the captured images displayed in a predetermined time lag. Furthermore, an inconvenience due to the partial display of the real-time images can be suppressed.

In the image display apparatus according to the second embodiment, the data expander 29 that performs expansion process on the compressed data is provided not to the receiving device 4, but to the image display apparatus itself. With such a configuration, the receiving device 4 has advantages that an increase in the power consumption due to the data expansion process can be avoided, resulting in the reduction in size and weight of the driving battery of the receiving device 4, and the burden of the subject 2 in carrying the receiving device 4 can be reduced.

The present invention is explained above with respect to the first and second embodiments, however, the present invention is not limited to the embodiments, and various embodiments or modifications can be conceivable by those skilled in the art. For example, the information recorded in the buffer memory 18 in the first and the second embodiments may not necessarily be the electric signal at a stage of demodulating process after the reception, and may be an electric signal generated as image data after the demodulating process. The real-time image and the past image may not necessarily be displayed at the same time, and only one of the real-time image and the past image may be displayed depending on the necessity. Moreover, a configuration other than the buffer memory for the mechanism which gives a predetermined time lag on the electric signal may be employed for displaying the past image.

### INDUSTRIAL APPLICABILITY

As described above, the image display apparatus according to the present invention is useful for a simple perusal of images, including a real-time display perusal of images captured by a body-insertable device, specifically suitable for an application in which a receiving process is performed on a radio signal transmitted from the capsule endoscope and the captured images are perused at the site.

## Claims

1. An image display apparatus, which is capable of sequentially displaying a plurality of images based on an electric signal sequentially generated via at least a receiving process on a predetermined radio signal, comprising:
a display unit that can display a real-time image as an image to be displayed via a sequential process in accordance with a generation of the electric signal, and a past image as an image to be displayed in a predetermined time lag after the generation of the electric signal.

2. The image display apparatus according to claim 1, further comprising:
a buffer memory unit that, after the generation of the electric signal, retains one of the electric signal and image data which is generated based on the electric signal for a predetermined time to output thereafter, wherein
the display unit displays the past time image based on one of the electric signal and the image data output from the buffer memory unit.

3. The image display apparatus according to claim 1 or 2, wherein the display unit displays the real-time image and the past image on the same screen at the same time.

4. The image display apparatus according to claim 3, wherein the display unit has a main image area and a subimage area which is smaller than the main image area on the display screen, and displays an image which is selected among the plurality of images displayed at the same time, in the main image area.

5. The image display apparatus according to any one of claims 1 to 4, wherein the electric signal is compressed data on which a predetermined compression process is performed, and
the image display apparatus further comprises:
a data expander that performs an expansion process with respect to the compressed data when image data is generated based on the electric signal.

6. The image display apparatus according to any one of claims 1 to 5, the display unit performs a partial display by appropriately thinning out the real-time images depending on a process speed in the image display apparatus, while all images corresponding to the electric signal are displayed with respect to the past image.
